# EUROPEAN PATENT APPLICATION

(11) **EP 4 306 527 A1**
(43) Date of publication of application: **17.01.2024**
(21) Application number: 22766594.0
(22) Date of filing: 14.01.2022
(51) Int. Cl.: C07D 519/00, A61K 9/14, A61K 31/4745, A61P 35/00, C07D 491/22

(54) **SN-38 DERIVATIVE, NANO-PARTICLES CONTAINING SAID DERIVATIVE, MEDICINE, AND METHOD FOR PRODUCING SAID NANO-PARTICLES**

(30) Priority: 12.03.2021 JP 2021040799
(71) Applicant: Japan Science and Technology Agency, Kawaguchi-shi, Saitama 332-0012 (JP)
(72) Inventor: KASAI, Hitoshi, Sendai-shi, Miyagi 980-8577 (JP); TANITA, Keita, Sendai-shi, Miyagi 980-8577 (JP); KOSEKI, Yoshitaka, Sendai-shi, Miyagi 980-8577 (JP)
(74) Representative: Gilani, Anwar
(86) International application number: PCT/JP2022/001163
(87) International publication number: WO 2022/190626

(57) **Abstract**

The problem to be solved by the present invention is to provide novel organic nanoparticles and a novel compound for constituting the organic nanoparticles. The present invention provides a compound represented by the following formula (1) or a salt thereof: 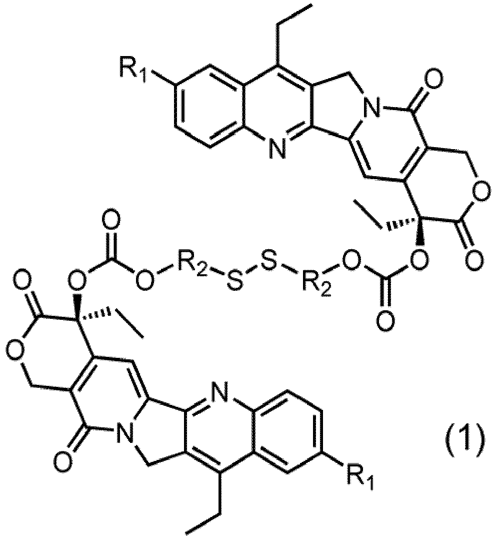 wherein R¹s are the same or different and each represent hydrogen, a hydroxy group, or a carbon-containing organic group; and R²s are the same or different and each represent an optionally substituted ethylene group or trimethylene group.

## Description

### Technical Field

### Cross-Reference to Related Application

The present application claims priority from Japanese Patent Application No. 2021-40799, filed on March 12, 2021, the entire disclosure of which is incorporated herein by reference. The present invention relates to an SN-38 derivative, nanoparticles containing the derivative, a medicine, and a method for producing the nanoparticles.

### Background Art

In recent years, many carrier-free nanopharmaceuticals have been developed in order to avoid various problems caused by carriers. Several methods are known, such as the top-down method, in which drug plasma is crushed and pulverized, and the bottom-up method, in which nanoparticles are prepared from molecules. Under these circumstances, the present inventors have developed a unique method for producing organic nanoparticles, the "reprecipitation method," and have established a technology for producing nanoparticles with a particle size of 100 nm or less (NPL 1 and PTL 1). Carrier-free nanopharmaceuticals obtained by these methods have been shown to have high pharmacological effects.

However, the development of novel organic nanoparticles and novel compounds for constituting the organic nanoparticles is still desired. For example, there is a strong need for organic nanoparticles capable of selective drug release in tumor tissue when used as antitumor agents.

### Citation List

### Patent Literature

PTL 1: JP2019-094260A

### Non-patent Literature

NPL 1: Jpn. J. Appl. Phys. 1992, 31, L1132-L1134

### Summary of Invention

### Technical Problem

An object of the present invention is to provide novel organic nanoparticles and a novel compound for constituting the organic nanoparticles. In a typical embodiment, an object of the present invention is to provide organic nanoparticles capable of selective drug release in tumor tissue.

### Solution to Problem

Under these circumstances, the present inventors focused on glutathione (GSH), which is a compound present in high concentration within cancer cells and acting as an electron donor to reduce disulfide bonds to cysteine, and tried to dimerize antitumor agent compounds by breaking SS bonds. More specifically, first, SN-38 represented by the following formula: 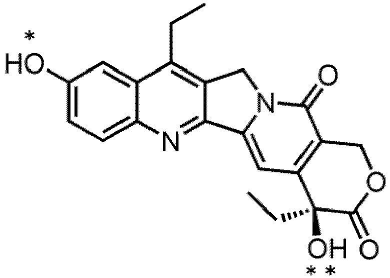 was selected from various compounds. Then, a compound was synthesized by linking together the primary hydroxy groups of SN-38 (the hydroxy group indicated by * in the above formula) via an S-S bond, and its performance was evaluated. As a result, there was a problem that the drug was released before it reached the affected area due to its rapid hydrolysis in the blood. Accordingly, next, based on a derivative in which the primary hydroxy group of SN-38 was replaced by C6 acyloxy, the following compound was synthesized by linking together the tertiary hydroxy groups of the derivatives (the hydroxy group indicated by ** in the above formula) via an S-S bond:

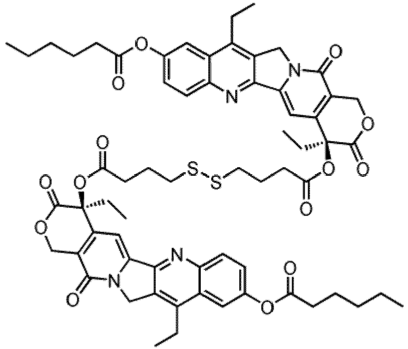

Then, nanoparticles were produced. As a result, the produced nano-prodrug showed good dispersion stability for 7 days or more. However, when dithiothreitol and glutathione (reduced form) were added to the nano-prodrug to confirm whether drug release triggered by the S-S bond was achieved, the cleavage of the S-S bond proceeded somewhat; however, the cleavage reaction was still in progress. Since the formation of C6 acyloxy-substituted SH-38 derivative was not confirmed, it is considered that the reaction to form C6 acyloxy-substituted SH-38 derivative and dihydrothiophen-2(3H)-one in such a manner that the group - CC(=O)-CH₂-CH₂-CH₂-S-SH formed by the cleavage of the S-S bond was eliminated to form a ring also did not proceed. Based on these results, the present inventors further examined various reaction systems, and as a result of intensive research, found that when SH-38 or a derivative thereof was dimerized via an S-S bond, the dissociation of SH-38 or the derivative thereof proceeded well by using a carbonate bond (-OC(=O)-O-) rather than an ester bond as in the above compound. The present invention is based on these new findings. Therefore, the present invention provides the following items:
Item 1. A compound represented by the following formula (1) or a salt thereof: 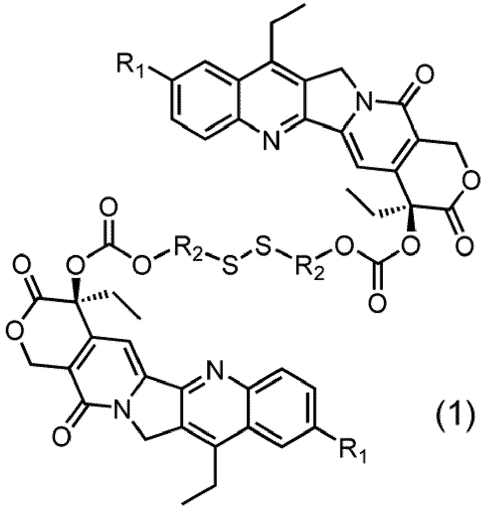 wherein R¹s are the same or different and each represent hydrogen, a hydroxy group, or a carbon-containing organic group; and R²s are the same or different and each represent an optionally substituted ethylene group or trimethylene group.
Item 2. The compound according to Item 1 or a salt thereof, which has a ClogP of 5.0 to 14.0.
Item 3. The compound according to Item 1 or 2 or a salt thereof, wherein R¹ is hydrogen, a hydroxy group, -O-C(=O)-L-R³, wherein L represents a single bond or a linker group, and R³ represents an optionally substituted unsaturated hydrocarbon ring group or heterocyclic group, or -O-C(=O)-(Y)n-R⁴, wherein Y represents a nitrogen atom or an oxygen atom, n represents 0 or 1, and R⁴ represents a C2-C10 alkyl group, a C3-C10 cycloalkyl group, a C4-C10 cycloalkyl-alkyl group, or a C4-C10 alkyl-cycloalkyl group.
Item 4. Nanoparticles comprising the compound according to any one of Items 1 to 3 or a salt thereof.
Item 5. A medicine comprising the compound according to any one of Items 1 to 3 or a salt thereof, or the nanoparticles according to Item 4.
Item 6. The medicine according to Item 5, for use in prevention or treatment of cancer.
Item 7. A method for producing nanoparticles, comprising injecting a water-miscible organic solvent solution of the compound according to any one of Items 1 to 3 or a salt thereof into water.
Item 8. A method for preventing or treating cancer, comprising administering an effective amount of the compound according to any one of Items 1 to 3 or a salt thereof, or the nanoparticles according to Item 4, to a subject in need thereof.
Item 9. Use of the compound according to any one of Items 1 to 3 or a salt thereof, or the nanoparticles according to Item 4, for producing a preventive or therapeutic agent for cancer.
Item 10. The compound according to any one of Items 1 to 3 or a salt thereof, or the nanoparticles according to Item 4, for use in prevention or treatment of cancer.

### Advantageous Effects of Invention

According to the present invention, organic nanoparticles capable of selective drug release in tumor tissue can be provided.

### Brief Description of Drawings

Fig. 1 shows the results of an in vitro activity test in Test Example 1.
Fig. 2 shows the SEM photograph of nanoparticles of compound B in Example 1.
Fig. 3 shows the results of selection of compounds used when Test Example 2 is performed.
Fig. 4 shows the SEM photographs of SNC4DC nanoparticles and the results of changes in particle size over time in Example 9.
Fig. 5 shows the results of an in vivo antitumor activity test in Test Example 2.
Fig. 6 shows changes in body weight over time in each test group in Test Example 2.

### Description of Embodiments

### Compound or Salt Thereof

The present invention provides a compound represented by the following formula (1) or a salt thereof: 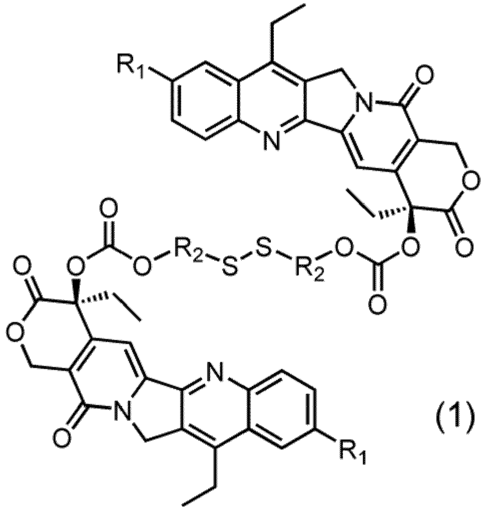 wherein R¹s are the same or different and each represent hydrogen, a hydroxy group, or a carbon-containing organic group; and R²s are the same or different and each represent an optionally substituted ethylene group or trimethylene group.

In the present invention, examples of the carbon-containing organic group include organic groups having 1 to 20 carbon atoms, and preferably 2 to 17 carbon atoms. The carbon-containing organic group may have at least one heteroatom selected from the group consisting of oxygen, sulfur, and nitrogen. More specific examples of the carbon-containing organic group include an alkyl group, an alkoxy group, -O-C(=O)-L-R³ (wherein L represents a single bond or a linker group, and R³ represents an optionally substituted unsaturated hydrocarbon ring group or heterocyclic group), -O-C(=O)-(Y)n-R⁴ (wherein Y represents a nitrogen atom or an oxygen atom, n represents 0 or 1, and R⁴ represents a C2-C10 alkyl group, a C3-C10 cycloalkyl group, a C4-C10 cycloalkyl-alkyl group, or a C4-C10 alkyl-cycloalkyl group), and the like. Examples of the carbon-containing organic group represented by formula -O-C(=O)-L-R³ include a 5-(1,2-dithiolan-3-yl)pentanoyloxy group and the like. Examples of the carbon-containing organic group represented by - O-C(=O)-(Y)n-R⁴ include acyloxy, cycloalkylcarbonyloxy, cycloalkylalkylcarbonyloxy, alkylcycloalkylcarbonyloxy, alkylcarbamoyloxy, and alkoxycarbonyloxy groups.

In the present invention, the "alkyl group" refers to a linear or branched saturated hydrocarbon group. Examples include C1-C10 alkyl groups, such as methyl, ethyl, n-propyl, isopropyl, n-butyl, isobutyl, sec-butyl, tert-butyl, n-pentyl, isopentyl, n-hexyl, n-heptyl, n-octyl, n-nonyl, and n-decyl groups; preferably C1-C8 alkyl groups etc., more preferably C1-C6 alkyl groups etc., and even more preferably C1-C3 alkyl groups etc.

In the present invention, the "alkoxy group" refers to an alkoxy group whose alkyl moiety is any of the above alkyl groups. More specific examples of the alkoxy group include C1-C10 alkoxy groups, such as methoxy, ethoxy, n-propoxy, isopropoxy, n-butoxy, isobutoxy, sec-butoxy, tert-butoxy, n-pentyloxy, isopentyloxy, n-hexyloxy, n-heptyloxy, n-octyloxy, n-nonyloxy, and n-decyloxy groups; preferably C1-C8 alkoxy groups etc., more preferably C1-C6 alkoxy groups etc., and even more preferably C1-C3 alkoxy groups etc.

In the present invention, the acyloxy group refers to a carbonyloxy group to which linear or molecular chained (preferentially linear) saturated hydrocarbon is attached. Examples include acetoxy, propionyloxy, butyryloxy, isobutyryloxy, pivaloyloxy, pentanoyloxy, hexanoyloxy, heptanoyloxy, octanoyloxy, nonanoyloxy, decanoyloxy, undecanoyloxy, and dodecanoyloxy groups. In the present invention, C2-C10 acyloxy groups are preferred, C4-C8 acyloxy groups are more preferred, and C4-C6 acyloxy groups are even more preferred. In the present invention, the "CX acyloxy group" refers to an acyloxy group having X number of carbon atoms, including the carbon atom constituting the carbonyl group. Therefore, for example, an acetoxy group (-O-C(=O)-CH₃) is a C2 acyloxy group, and an undecanoyloxy group (-O-C(=O)-CH₂-CH₂-CH₂-CH₂-CH₂-CH₂-CH₂-CH₂-CH₂-CH₃) is a C11 acyloxy group.

In the present invention, the "cycloalkyl group" refers to a cyclic hydrocarbon group. Examples include C3-C10 alkyl groups, such as cyclopropyl, cyclobutyl, cyclopentyl, cyclohexyl, cycloheptyl, cyclooctyl, cyclononyl, and cyclosyl groups; preferably C3-C8 alkyl groups etc., more preferably C3-C6 alkyl groups etc., and even more preferably C3-C4 alkyl groups etc.

In the present invention, the "cycloalkyl-alkyl group" refers to an alkyl group substituted with one cycloalkyl group. Examples of the "cycloalkyl-alkyl group" include cycloalkyl-alkyl groups whose cycloalkyl moiety is any of the above cycloalkyl groups and whose alkyl moiety is any of the above alkyl groups. In the present invention, C4-C10 cycloalkyl-alkyl groups are preferred, and C4-C6 cycloalkyl-alkyl groups are more preferred. In the present invention, the "C4-C10 cycloalkyl-alkyl group" refers to a cycloalkyl-alkyl group whose cycloalkyl and alkyl moieties have a total of 4 to 10 carbon atoms. More specific examples of the cycloalkyl-alkyl group include cyclopropylmethyl, cyclobutylmethyl, cyclohexylmethyl, cyclononylmethyl, 1-cyclopropylethyl, 2-cyclopropylethyl, 3-cyclopropylpropyl, 6-cyclopropylhexyl, and the like; and preferably cyclopropylmethyl etc.

In the present invention, the "alkyl-cycloalkyl group" refers to a cycloalkyl group substituted with one alkyl group. Examples of the "alkyl-cycloalkyl group" include alkyl-cycloalkyl groups whose alkyl moiety is any of the above alkyl groups, and whose cycloalkyl moiety is any of the above cycloalkyl groups. In the present invention, C4-C10 alkyl-cycloalkyl groups are preferred, and C4-C6 alkyl-cycloalkyl groups are more preferred. In the present invention, the "C4-C10 alkyl-cycloalkyl group" refers to an alkyl-cycloalkyl group whose alkyl and cycloalkyl moieties have a total of 4 to 10 carbon atoms. More specific examples of alkyl-cycloalkyl groups include 1-methylcyclopropyl, 2-methylcyclopropyl, 1-methylcyclobutyl, 2-methylcyclohexyl, 4-methylcyclohexyl, 5-methylcyclononyl, 2-ethylcyclopropyl, 2-propylcyclopropyl, 2-hexylcyclopropyl, and the like; and preferably 1-methylcyclopropyl, 2-methylcyclopropyl, and the like.

In formula (1), R¹s may be the same or different, but are preferably the same.

In formula (1), the ethylene group or trimethylene group represented by R² may be unsubstituted or substituted. When the ethylene group or trimethylene group represented by R² has a substituent, the substituent is not particularly limited, and examples include alkyl groups (e.g., C1-C10 alkyl groups) and the like. When the ethylene group or trimethylene group represented by R² has a substituent, the number of substituents is not limited. For example, each R² preferably has one or two substituents. When each R² has two substituents, the two substituents are preferably bonded to one carbon of the methylene groups that constitute the ethylene group or trimethylene group.

In the present invention, the linker group refers to, for example, -O-C(=O)-L¹-L²-, -O-C(=S)-L¹-L²-, -O-C(=O)-O-L¹-L²-, - O-C(=O)-NH-L¹-L²-, or -O-C (=S) -NH-L¹-L²- (wherein L¹ represents a single bond or a divalent linear C1-6 (preferably C3-5) saturated hydrocarbon group, and L² represents a single bond or -O-C(=O)-). Such a linker group binds to -O-C(=O)- by the bond on the left side in the above notation and to R³ by the bond on the right side. For example, when the linker group is a group -O-C(=O)-L¹-L²-, the group -O-C(=O)-L¹-L²- binds to -O-C(=O)- by the bond on the oxygen side and to R³ on the L² side. When L² is -O-C(=O)-, it binds to L¹ by the bond on the -O- side and to R³ by the bond on the -C(=O)- side.

In the present invention, the "unsaturated hydrocarbon ring group" refers to a hydrocarbon ring group having at least one double bond (preferably two or more double bonds). Examples include aryl and non-aromatic unsaturated hydrocarbon ring groups, and preferably non-aromatic unsaturated hydrocarbon ring groups. Examples of unsaturated hydrocarbon ring groups include those having 5 to 10 carbon atoms. The number of double bonds in the unsaturated hydrocarbon ring group is also not limited, but is, for example, 1 to 5, and preferably 2 to 4.

In the present invention, the "aryl group" refers to a monovalent group obtained by removing one hydrogen from the aromatic ring of an aromatic hydrocarbon, and examples include C6-10 monocyclic or bicyclic aryl groups, such as naphthyl group.

In the present invention, the "non-aromatic unsaturated hydrocarbon ring group" refers to an unsaturated hydrocarbon ring group other than aryl groups, and examples include C5-10 (preferably C6-8) monocyclic or bicyclic (preferably monocyclic) unsaturated hydrocarbon ring groups. The number of double bonds in the non-aromatic unsaturated hydrocarbon ring group is also not limited, but is, for example, 1 to 4, and preferably 2 or 3. More specific examples include cyclopentenyl, cyclopenta-2,4-dienyl, cyclohexa-2,4-dienyl, cyclohepta-2,4,6-trienyl, 1H-indenyl, and 1,2-dihydronaphthyl groups.

In the present invention, examples of the "heterocyclic group" include monocyclic or bicyclic (preferably monocyclic) saturated or unsaturated (preferably saturated) heterocycles having 1 to 9 (preferably 1 to 7, more preferably 2 to 5, and even more preferably 2 to 4) carbon atoms and having at least one heteroatom (preferably 1 to 3 heteroatoms, and more preferably 2 heteroatoms) selected from the group consisting of oxygen, sulfur, and nitrogen. The heteroatom contained in the heterocycle is preferably sulfur. The heterocycle is, for example, preferably a 4- to 10-membered ring, and more preferably a 4- to 6-membered ring. More specific examples of heterocycles include dithietanyl, dithiolanyl, tetrahydrofuranyl, tetrahydrothiophenyl, tetrazolidinyl, pyrrolidinyl, morpholinyl, pyridinyl, piperidinyl, dithianyl, furanyl, thiophenyl, and quinolinyl groups.

In the present invention, the unsaturated hydrocarbon ring group and/or heterocyclic group is optionally substituted. Examples of substituents include alkyl, oxo, hydroxy, alkoxy, amino, amine, and halogen groups; and preferably alkyl and oxo groups. In this embodiment, when the unsaturated hydrocarbon ring group or heterocyclic group has multiple substituents, those substituents may be the same or different. When the unsaturated hydrocarbon ring group and/or heterocyclic group has a substituent, the number of substituents is not limited, and is at least 1, 1 to 3, 1 or 2, or the like.

In an embodiment of the present invention in which R¹ is an optionally substituted unsaturated hydrocarbon ring group, the "optionally substituted unsaturated hydrocarbon ring group" is preferably aromatic (electrons are delocalized as in a resonance structure). Therefore, in this embodiment, preferred examples of the "optionally substituted unsaturated hydrocarbon ring group" include aryl groups, aromatic aryl groups having a substituent, and the like. Other preferred examples of the "optionally substituted unsaturated hydrocarbon ring group" include those in which one or more hydrogen atoms in a non-aromatic unsaturated hydrocarbon ring group are replaced by the above substituents, and which become aromatic.

The unsaturated hydrocarbon ring group having a substituent is preferably a 5-isopropyl-7-oxocyclohepta-1,3,5-trienyl group or the like.

In the present invention, the "amine group" refers to an amino group having one or two alkyl groups, aryl groups, or the like as substituents. When the amine group has two of the above substituents, the two substituents may be the same or different.

In the present specification, the compound represented by formula (1) or a salt thereof is also simply referred to as compound (1). In a typical embodiment of the present invention, compound (1) is used as an antitumor agent, more specifically as a prodrug of an antitumor agent. Therefore, in the present invention, compound (1) is preferably prevented from being hydrolyzed in the blood (release of the active ingredient from the prodrug molecule due to the cleavage of the carbonate moiety), and is preferably hydrolyzed in tumor cells to produce the active ingredient SN-38 or a derivative thereof. In the present invention, in terms of hydrolysis resistance in the blood, dispersion stability required for the production of nanoparticles, selective drug release within cancer cells, and the like, compound (1) preferably has a ClogP of 5.0 to 14.0, and more preferably 7.0 to 12.0. ClogP is the octanol/water partition coefficient logP calculated from the structural formula of the target compound using the ClogP program of ChemDraw Professional 16.0.

Table 1 below shows the ClogP values of typical compounds of the present invention.

**Table 1**

| Compound | ClogP |
|---|---|
| 7-Ethyl-camptothecin dimer (S-S, carbonate) | 5.6 |
| SN-38 dimer (S-S, carbonate) | 5.7 |
| SN-38xC₂ dimer (S-S, carbonate) | 5.0 |
| SN-38xC₄ dimer (S-S, carbonate) | 7.1 |
| SN-38xC₆ dimer (S-S, carbonate) | 9.2 |
| SN-38xC₈ dimer (S-S, carbonate) | 11.3 |
| SN-38xC₁₀ dimer (S-S, carbonate) | 13.4 |

In the above table, "SN-38 dimer (S-S, carbonate)" refers to a compound of formula (1) wherein R¹s are both hydroxy groups, and R²s are both ethylene groups. In the above table, "SN-38xCₐ dimer (S-S, carbonate)" refers to a compound of formula (1) wherein R¹s are both acyloxy groups with carbon number a, and R²s are both ethylene groups. For example, SN-38xC₂ dimer (S-S, carbonate) refers to a compound of formula (1) wherein R¹s are both acetoxy groups, and R²s are both ethylene groups.

Further, specific examples of compound (1) include dimers of the following groups:

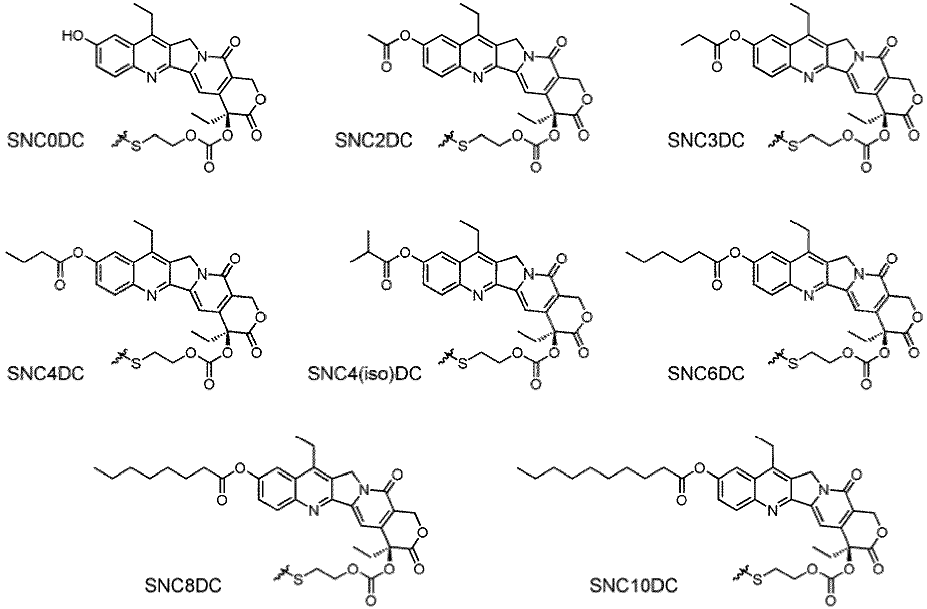

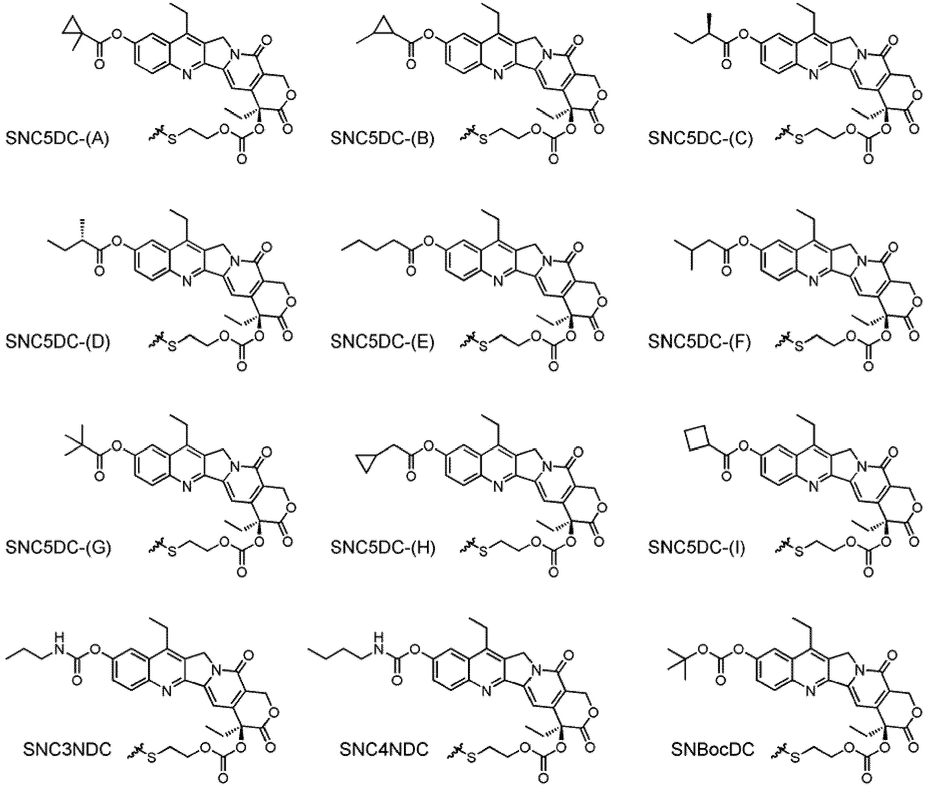

In the present invention, salts refer to pharmaceutically acceptable salts. Further, salts of compound (1) include acid addition salts and salts with bases. Specific examples of acid addition salts include inorganic acid salts, such as hydrochloride, hydrobromide, hydroiodide, sulfate, perchlorate, and phosphate; organic acid salts, such as oxalate, malonate, succinate, maleate, fumarate, lactate, malate, citrate, tartrate, benzoate, trifluoroacetate, acetate, methanesulfonate, p-toluenesulfonate, and trifluoromethanesulfonate; and acidic amino acid salts, such as glutamate and aspartate. Specific examples of salts with bases include alkali metal or alkaline earth metal salts, such as sodium, potassium, and calcium salts; salts with organic bases such as pyridine and triethylamine salts; salts with basic amino acids, such as lysine and arginine; and the like.

Since compound (1) or a salt thereof may exist in the form of a hydrate or solvate, the hydrate and solvate are also included in the compound that is the active ingredient of the present invention. When there are isomers, such as geometric isomers, stereoisomers, and optical isomers, of the compound of the present invention, these isomers are included in the compound of the present invention, unless otherwise specified.

Solvents that form solvates include water, alcohols such as ethanol and propanol, organic acids such as acetic acid, esters such as ethyl acetate, ethers such as tetrahydrofuran and diethyl ether, ketones such as acetone, DMSO, and the like.

Compound (1) can be produced by various methods, for example, the methods shown in the following reaction formulas. 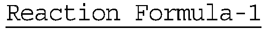 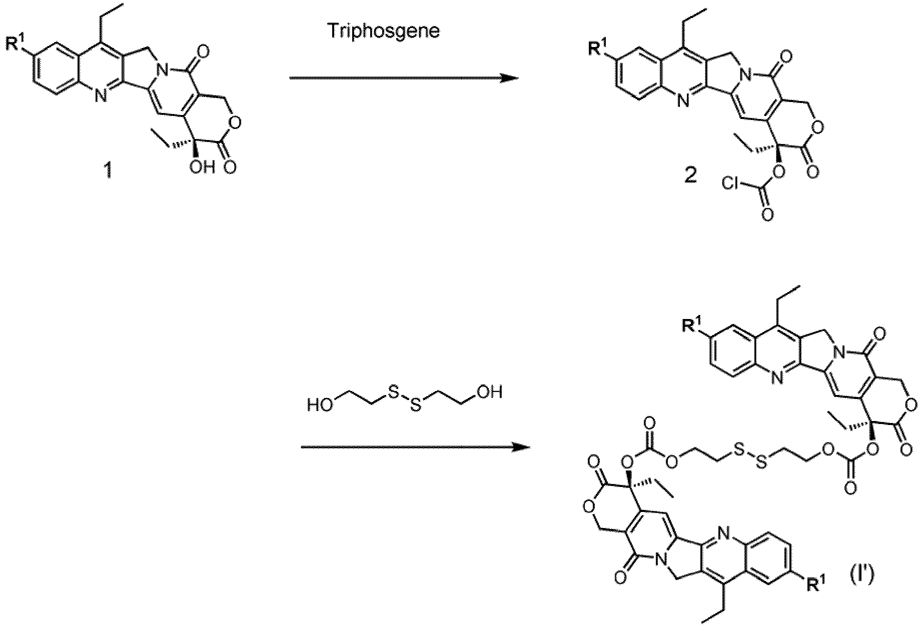 wherein R¹ is as defined above.

In reaction formula-1, first, compound 1 can be reacted with triphosgene to obtain compound 2. The use ratio of compound 1 and triphosgene is not particularly limited. For example, 0.3 to 0.4 mol of the latter can be used per one mol of the former. This reaction can be performed in the presence of a base, such as 4-dimethylaminopyridine (DMAP), triethylamine (TEA), or pyridine. The reaction temperature of the reaction is not particularly limited. The reaction is generally performed at room temperature or under cooling or heating. The reaction temperature is preferably 0 to 25°C or the like. The reaction time of the reaction is also not particularly limited, but can be, for example, 5 to 1 hour.

Next, compound 2 can be reacted with 2,2'-disulfanediylbis(ethan-1-ol) to obtain compound (1'). The use ratio of compound 2 and 2,2'-disulfanediylbis(ethan-1-ol) is not particularly limited. For example, 0.4 to 0.5 mol of the latter can be used per one mol of the former. The reaction temperature of the reaction is not particularly limited. The reaction is generally performed at room temperature or under cooling or heating. The reaction temperature is preferably 0 to 25°C or the like. The reaction time of the reaction is also not particularly limited, but can be, for example, 60 minutes to 24 hours.

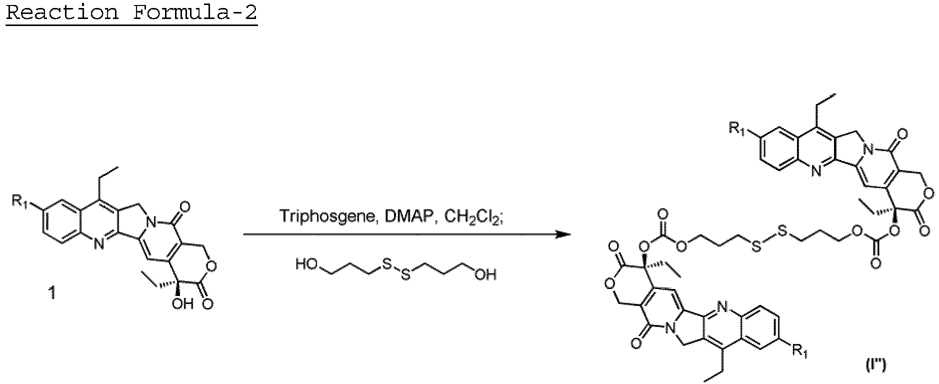

In reaction formula-2, compound 2 can be reacted with 2,2'-disulfanediylbis(propan-1-ol) to obtain compound (1''). The use ratio of compound 1 and 2,2'-disulfanediylbis(propan-1-ol) is not particularly limited. For example, 0.4 to 0.5 mol of the latter can be used per one mol of the former. The reaction temperature of the reaction is not particularly limited. The reaction is generally performed at room temperature or under cooling or heating. The reaction temperature is preferably 0 to 25°C or the like. The reaction time of the reaction is also not particularly limited, but can be, for example, 60 minutes to 24 hours.

These reactions are generally performed in conventional solvents that do not adversely affect the reactions, such as dichloromethane, triethylamine, acetone, methyl ethyl ketone, tetrahydrofuran, dioxane, diethyl ether diglyme, acetonitrile, N,N-dimethylformamide, dimethyl sulfoxide, and mixed solvents thereof.

### Nanoparticles

The present invention provides nanoparticles comprising compound (1) or a salt thereof. In the present invention, the nanoparticles refer to particles having an average particle size of less than 1 um. In a typical embodiment, the nanoparticles of the present invention are roughly spherical. In the present invention, the average particle size can be measured by scanning electron microscopy (SEM) or dynamic light scattering (DLS). The average particle size of the nanoparticles of the present invention is preferably 10 to 500 nm, and more preferably 10 to 100 nm. The nanoparticles of the present invention can be typically prepared by a method described later in which a water-miscible organic solvent solution of compound (1) or a salt thereof is injected into water and dispersed. Therefore, in a typical embodiment, the nanoparticles of the present invention are organic nanocrystals of compound (1) or a salt thereof, and thus substantially consist of compound (1) or a salt thereof. However, the nanoparticles of the present invention may contain components other than compound (1) or a salt thereof (e.g., additives for the water-miscible organic solvent used in the production of the nanoparticles) within the range in which the effects of the present invention can be obtained. For example, the content of compound (1) or a salt thereof in the nanoparticles of the present invention is preferably 95 mass% or more, more preferably 99 mass% or more, and even more preferably 99.9 mass% or more.

The nanoparticles of the present invention can be produced by a method comprising injecting a water-miscible organic solvent solution of compound (1) or a salt thereof into water. In the method of the present invention, first, a water-miscible organic solvent solution of compound (1) or a salt thereof is injected into water using a syringe. The water-miscible organic solvent is not particularly limited as long as it is a good solvent for compound (1) or a salt thereof. Examples include tetrahydrofuran, acetone, dioxane, acetonitrile, methanol, ethanol, propanol, N-methylpyrrolidone, dimethylsulfoxide, and the like. In terms of solubility and safety, acetone, tetrahydrofuran, ethanol, dimethylsulfoxide, and the like are preferred. These solvents can be used singly or as a mixture of two or more. The content of compound (1) or a salt thereof in the water-miscible organic solvent solution of compound (1) or a salt thereof is not particularly limited, but can be suitably set within the range of, for example, 0.1 to 15 mass%, and preferably 0.1 to 10 mass%. The water-miscible organic solvent solution may contain polysorbate 80 (PS80), polyvinylpyrrolidone (PVP), and the like, in addition to compound (1) or a salt thereof. The amount of the water-miscible organic solvent solution of compound (1) or a salt thereof to be injected into water is also not particularly limited. For example, 0.1 to 1 ml, and preferably 0.1 to 0.2 ml, of the water-miscible organic solvent solution can be added to 10 ml of water. The injection time is not particularly limited, but is preferably, for example, 0.1 to 1 second, and more preferably 0.1 to 0.2 seconds. The temperature of the reaction system in this step is also not particularly limited, but can be suitably set, for example, 0 to 30°C, and preferably 10 to 20°C. In an embodiment of the present invention, injection of the water-miscible organic solvent solution into water is preferably followed by stirring. The stirring speed is not particularly limited, but can be suitably set within the range of, for example, 1000 to 1500 rpm, and preferably 1200 to 1500 rpm. The temperature in the stirring step is the same as that in the injection step. The stirring time is not particularly limited, but can be suitably set within the range of, for example, 1 to 10 seconds, and preferably 1 to 3 seconds. By injecting the water-miscible organic solvent solution into water and optionally stirring, compound (1) or a salt thereof is crystallized or granulated. As a result, an aqueous dispersion of nanoparticles of compound (1) or a salt thereof can be obtained. In an embodiment of the present invention, the obtained nanoparticles can be used as a dispersion in a water-dispersed state. When the nanoparticles are used as a dispersion, it is preferable, in terms of safety, to remove the water-miscible organic solvent used for dissolving and adding compound (1) or a salt thereof to water, before use. The method for removing the organic solvent is not particularly limited, and known methods can be used. For example, the organic solvent can be removed by distillation under reduced pressure (or normal pressure), dialysis, or the like. In another embodiment of the present invention, the obtained nanoparticles can be isolated and used as fine particle powder by performing solid-liquid separation operation, such as filtration, on the dispersion. When the above production method is used, for example, the method can be performed with reference to the disclosure of PTL 1, except for using compound (1) or a salt thereof as a raw material. It is preferable that the compound or a salt thereof of the present invention is formed into nanoparticles as described above because a high-concentration dispersion (e.g., 0.1 to 10 mM, and preferably 1 to 10 mM of dispersion) can be obtained.

### Medicine

The present invention provides a medicine comprising compound (1) or a salt thereof. The present invention also provides a medicine comprising the nanoparticles of the present invention described above.

In the present invention, compound (1) or a salt thereof may be used as a medicine as it is, or may be used as a medicinal composition combined with various pharmaceutically acceptable carriers (e.g., tonicity agents, chelating agents, stabilizers, pH adjusters, preservatives, antioxidants, solubilizes, and thickening agents).

The medicine of the present invention can be used for prevention or treatment of cancer. In this embodiment, the cancer to be prevented or treated is not particularly limited, but is preferably solid tumor. Specific examples include esophageal cancer, gastric cancer, colon cancer, bowel cancer, rectal cancer, pancreatic cancer, liver cancer, laryngeal cancer, lung cancer, prostate cancer, bladder cancer, breast cancer, uterine cancer, and ovarian cancer. Target sites include tumor cells, tissues, organs or viscera, and their interiors.

Examples of tonicity agents include saccharides, such as glucose, trehalose, lactose, fructose, mannitol, xylitol, and sorbitol; polyhydric alcohols, such as glycerol, polyethylene glycol, and propylene glycol; inorganic salts, such as sodium chloride, potassium chloride, and calcium chloride; and the like.

Examples of chelating agents include edetates, such as disodium edetate, disodium calcium edetate, trisodium edetate, tetrasodium edetate, and calcium edetate; ethylenediaminetetraacetate, nitrilotriacetic acid or a salt thereof, sodium hexametaphosphate, citric acid, and the like.

Examples of stabilizers include sodium bisulfite and the like.

Examples of pH adjusters include acids, such as hydrochloric acid, carbonic acid, acetic acid, and citric acid; as well as alkali metal hydroxides, such as sodium hydroxide and potassium hydroxide; alkali metal carbonates or hydrogen carbonates, such as sodium carbonate; alkali metal acetates, such as sodium acetate; alkali metal citrates, such as sodium citrate; bases, such as trometamol; and the like.

Examples of preservatives include sorbic acid, potassium sorbate; paraoxybenzoic acid esters, such as methyl parahydroxybenzoate, ethyl parahydroxybenzoate, propyl parahydroxybenzoate, and butyl parahydroxybenzoate; chlorhexidine gluconate; quaternary ammonium salts, such as benzalkonium chloride, benzethonium chloride, and cetylpyridinium chloride; alkylpolyaminoethylglycine, chlorobutanol, polyquad, polyhexamethylene biguanide, chlorhexidine, and the like.

Examples of antioxidants include sodium bisulfite, dried sodium sulfite, sodium pyrosulfite, concentrated mixed tocopherols, and the like.

Examples of solubilizes include sodium benzoate, glycerol, D-sorbitol, glucose, propylene glycol, hydroxypropyl methylcellulose, polyvinylpyrrolidone, macrogol, D-mannitol, and the like.

Examples of thickening agents include polyethylene glycol, methylcellulose, ethylcellulose, carmellose sodium, xanthan gum, sodium chondroitin sulfate, hydroxyethylcellulose, hydroxypropylcellulose, hydroxypropylmethylcellulose, polyvinylpyrrolidone, polyvinyl alcohol, and the like.

In addition to compound (1) or a salt thereof, the medicinal composition may further contain compounds that are known to have antitumor effects. Examples of compounds that are known to have antitumor effects include chemotherapeutic agents, such as podophyllotoxin (PPT), doxorubicin (DOX), and fluorouracil (5-FU).

In an embodiment of the medicinal composition, the content of compound (1) or a salt thereof in the composition is not particularly limited, and can be set as appropriate, in terms of the content of compound (1), from conditions such as 90 mass% or more, 70 mass% or more, 50 mass% or more, 30 mass% or more, 10 mass% or more, 5 mass% or more, and 1 mass% or more.

The preparation form is not particularly limited, and examples include orally administered drugs, such as tablets, pills, capsules, powders, granules, syrups, and sublingual agents; parenterally administered drugs, such as injections (for intravenous injection, intramuscular injection, local injection, etc.), mouthwash, drops, external preparations (ointments, creams, patches, and inhalants), and suppositories; and other various preparation forms. Preferred among these preparation forms are, for example, injections (for intravenous injection), drops, and the like. Further, in a preferred embodiment of the present invention, compound (1) or a salt thereof is also useful because it exhibits an antitumor effect when directly injected into a tumor.

The content of compound (1) of the present invention in the preparation varies depending on the route of administration, patient age, body weight, symptoms, etc., and cannot be categorically defined; however, the daily dose of compound (1) may be generally about 10 to 5000 mg, and more preferably about 100 to 1000 mg. When administered once a day, one preparation may contain this amount, and when administered three times a day, one preparation may contain one-third of this amount.

The medicine of the present invention is administered to patients such as mammals. Examples of mammals include humans, monkeys, mice, rats, rabbits, cats, dogs, pigs, cows, horses, sheep, and the like.

### Examples

The present invention is described more specifically and in detail below while showing Examples and Comparative Examples; however, it should not be construed that the Examples limit the scope of the present invention.

### Comparative Example 1

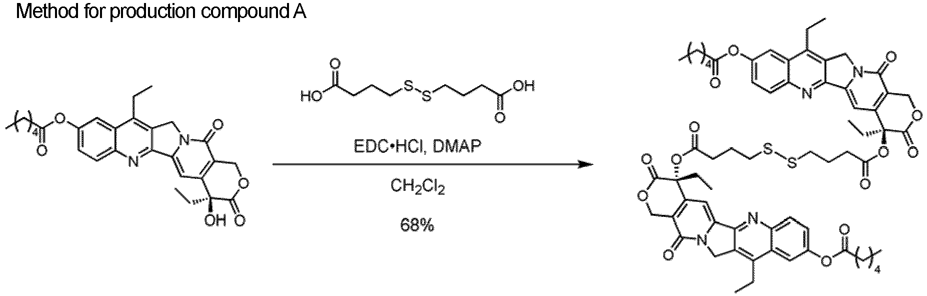

SN-38×C6 (106.2 mg, 0.216 mmol), 4,4'-disulfanediyldibutyric acid (27.9 mg, 0.117 mmol), 1-ethyl-3-(3-dimethylaminopropyl)carbodiimide hydrochloride (175.1 mg, 0.913 mmol), and 4-dimethylaminopyridine (9.1 mg, 0.0745 mmol) were placed in an eggplant flask equipped with a stirrer bar, and dissolved in 2.2 mL of dichloromethane. After stirring at room temperature overnight, the solvent was distilled off under reduced pressure, and the resulting residue was purified by silica gel column chromatography (chloroform ---> chloroform/methanol = 50:1), thereby obtaining compound A (87.1 mg, 68%) as a pale yellow solid.
¹H-NMR (CDCl₃, 400 MΣ3z) : δ = 0.956 (6H, t, J = 7 Hz), 1.41 (7H, m), 1.77-1.88 (4H, m), 2.1 (1H, sext, J= 7.2 Hz), 2.24 (1H, sext, J = 7.2 Hz), 2.51 (2H, m), 2.63 (4H, m), 3.13 (2H, q, J = 7.7 Hz), 5.23 (2H, s), 5.37 (1H, d, J = 17 Hz), 5.7 (1H, d, J = 17 Hz), 7.12 (1H, s), 7.55 (1H, dd, 9.2, 2.4)

### Example 1

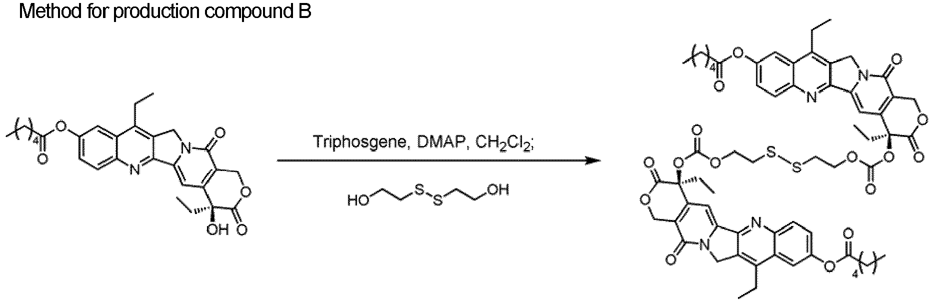

SN-38×C6 (50 mg, 0.1 mmol), triphosgene (12 mg, 0.04 mmol), and 4-dimethylaminopyridine (61 mg, 0.5 mmol) were placed in an eggplant flask equipped with a stirrer bar, and dissolved in 2 mL of dichloromethane. After stirring at room temperature for 15 minutes, 2,2-dithiodiethanol dissolved in 1 mL of dichloromethane was added dropwise. After stirring at room temperature overnight, the resultant was diluted with chloroform and washed with water and a saturated sodium chloride solution. The organic layer was dried over anhydrous magnesium sulfate, followed by filtration and distillation of the solvent under reduced pressure. The resulting residue was purified by silica gel column chromatography (hexane/ethyl acetate = 3:1 ---> ethyl acetate), thereby obtaining compound B (22 mg, 46%) as a pale yellow solid. In the present specification, compound B is also referred to as compound SNC6DC.
¹H-NMR (CDCl₃, 400 MΣ3z) : δ = 0.956 (6H, t, J = 7 Hz), 1.38-1.44 (7H, m), 1.82 (2H, quin, J = 5.6 Hz), 2.05-2.11 (1H, m), 2.17-2.22 (1H, m), 2.66 (2H, t, J = 7.4 Hz), 2.78 (2H, t, J = 6.2 Hz), 3.15 (2H, q, J = 7.7 Hz), 3.88-4.00 (2H, m), 5.26-5.32 (2H, m), 5.35 (1H, d, J = 17 Hz), 5.8 (1H, d, J = 17 Hz), 7.21 (1H, s), 7.55 (1H, dd, J = 9.2, 2.4 Hz), 7.82 (1H, d, J = 2.4 Hz), 8.17 (1H, d, J = 9.2 Hz).

### Example 2

### Method for producing nanoparticles of compounds A and B of the present invention

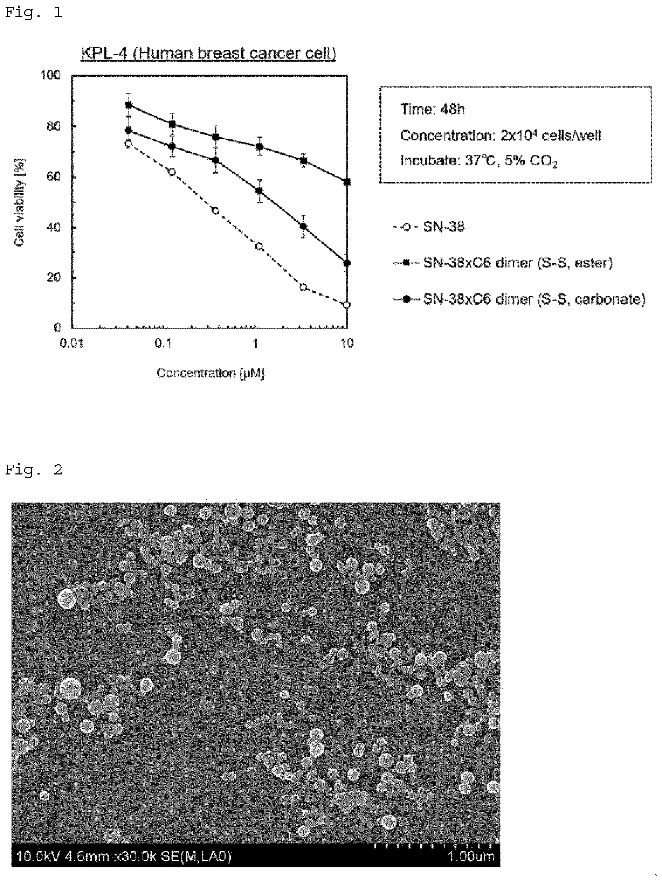

100 µL of THF solution of compound A or B prepared to 10 mM was injected into 10 mL of water using a syringe at room temperature, followed by stirring for 2 seconds at 1500 rpm, thereby obtaining an aqueous dispersion of nanoparticles. The concentration of the final aqueous dispersion was 0.1 mM. SEM and DLS revealed that the particle size was about 100 nm. Fig. 2 shows the SEM of the nanoparticles of compound B obtained above.

### Test Example 1

### In vitro activity test

Human liver cancer cell line HePG2 or human breast cancer cell line KPL-4 was seeded in a 96-well plate at 2×10⁴ cells/well. On the next day, irinotecan and each SN-38 derivative (compound A ((SN-38xC₆ dimer (S-S, ester)), compound B (SN-38xC₆ dimer (S-S, carbonate))) nanoparticle dispersion were added to the HepG2 cell culture medium to 0.04 to 10 µM. Then, the cells were cultured for 48 hours, and the cell viability was measured by a colorimetric method using the Cell Counting Kit-8 (produced by Dojindo Laboratories) and a microplate reader.

Fig. 1 shows the results. As shown in Fig. 1, compound A did not exhibited good pharmacological activity, while compound B exhibited good pharmacological activity due to the drug derived from the cleavage of the -S-S- bond.

### Example 3

### Method for producing compound SNBocDC of the present invention

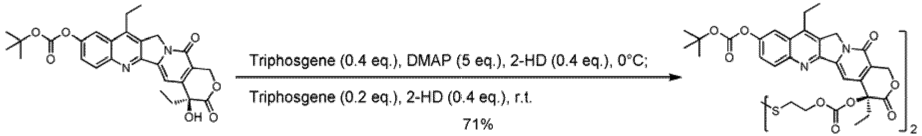

Boc-SN-38 (1476 mg, 3.00 mmol), triphosgene (356 mg, 1.20 mmol), 4-dimethylaminopyridine (1830 mg, 15.0 mmol), and 2-hydroxyethyl disulfide (193 mg, 1.25 mmol) were placed in an eggplant flask equipped with a stirrer bar, and dissolved in 50 mL of dichloromethane at 0°C. After stirring at 0°C for 1 hour, triphosgene (178 mg, 0.600 mmol) and 2-hydroxyethyl disulfide (193 mg, 1.25 mmol) were placed therein. After stirring at room temperature for 2 hours, the resultant was diluted with chloroform and washed with water and a saturated sodium chloride solution. The organic layer was dried over anhydrous magnesium sulfate, followed by filtration and distillation of the solvent under reduced pressure. The resulting residue was recrystallized with methanol and filtered, thereby obtaining compound SNBocDC (1269 mg, 71%) as a pale yellow solid.
¹H-NMR (CDCl₃, 400 MΣ3z) : δ = 0.949 (3H, t, J = 7.4 Hz), 1.39 (3H, t, J = 7.8 Hz), 1.60 (9H, s), 2.03-2.23 (2H, m), 2.77 (2H, t, J = 6.2 Hz), 3.15 (q, J = 7.7 Hz), 3.85-4.00 (2H, m), 5.25 (2H, d, J = 5.6 Hz), 5.34 (1H, d, J = 17.2 Hz), 5.80 (1H, d, J = 17.2 Hz), 7.20 (1H, s), 7.65 (1H, dd, J = 9.2, 2.4 Hz), 7.89 (1H, d, J = 2.4 Hz), 8.16 (1H, d, J = 9.2 Hz).

### Example 4

### Method for producing compound SNC0DC of the present invention

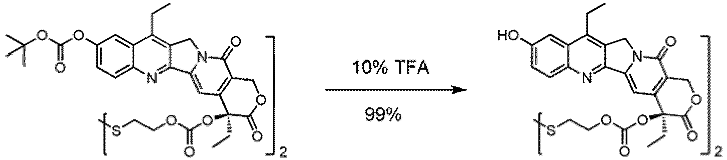

SNBocDC (723 mg, 0.607 mmol) was placed in an eggplant flask equipped with a stirrer bar, and dissolved in 22 mL of dichloromethane. 2.2 mL of trifluoroacetic acid was placed therein, the mixture was stirred at room temperature for 3.5 hours, and the solvent was then distilled off under reduced pressure. The resulting residue was recrystallized with chloroform and filtered, thereby obtaining compound SNC0DC (596 mg, 99%) as a pale yellow solid.
¹H-NMR (DMSO, 400 MHz): δ = 0.881 (3H, t, J = 7.4 Hz), 1.26 (3H, t, J = 7.6 Hz), 2.08-2.15 (2H, m), 2.90-2.99 (2H, m), 3.04 (2H, q, J = 7.5 Hz), 5.23 (2H, s). 5.5 (2H, d, J = 3.2 Hz), 6.91 (1H, s). 7.36-7.38 (2H, m), 7.96 (1H, d, J = 10 Hz), 10.3 (1H, br).

### Example 5

### Method for producing compound SNC2DC of the present invention

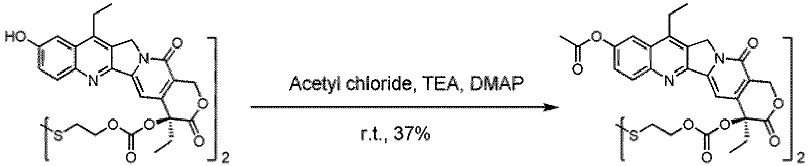

SNC0DC (200 mg, 0.202 mmol), triethylamine (101 mg, 0.998 mmol), and 4-dimethylaminopyridine (5 mg, 0.0409 mmol) were placed in an eggplant flask equipped with a stirrer bar, and dissolved in 2.8 mL of dichloromethane at 0°C. 36 µL of acetyl chloride was placed therein, the mixture was stirred at room temperature overnight, and the solvent was then distilled off under reduced pressure. The resulting residue was diluted with chloroform and washed with water and a saturated sodium chloride solution. The organic layer was dried over anhydrous magnesium sulfate, followed by filtration and distillation of the solvent under reduced pressure. The resulting residue was purified by silica gel column chromatography (chloroform ---> chloroform/acetone = 9:1), thereby obtaining compound SNC2DC (80.8 mg, 37%) as a pale yellow solid.
¹H-NMR (CDCl₃, 400 MHz): δ = 0.953 (3H, t, J = 7.4 Hz), 1.37 (3H, t, J = 7.6 Hz), 2.03-2.25 (2H, m), 2.40 (3H, s), 2.78 (2H, t, J = 6.2 Hz), 3.14 (2H, q, J = 7.7 Hz), 3.87-4.02 (2H, m), 5.25 (2H, d, J = 5.2 Hz), 5.34 (1H, d, J = 17.2 Hz), 5.78 (1H, d, J = 16.8 Hz), 7.20 (1H, s), 7.55 (1H, dd, J = 9.2, 2.8 Hz), 7.82 (1H, d, J = 2.4 Hz), 8.16 (1H, d, J = 9.2 Hz).

### Example 6

### Method for producing compounds SNC3DC, SNC4DC, SNC8DC, SNC10DC, and SNC5DC-(A), (B), (C), (D), (E), (F), (G), (H), and (I) of the present invention

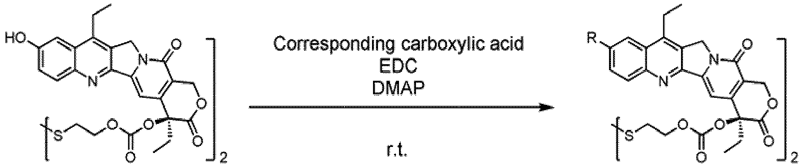

First, the production method is described using SNC3DC as an example.

SN0DC (200 mg, 0.202 mmol), 1-ethyl-3-(3-dimethylaminopropyl)carbodiimide hydrochloride (78 mg, 0.502 mmol), and 4-dimethylaminopyridine (5 mg, 0.0409 mmol) were placed in an eggplant flask equipped with a stirrer bar, and dissolved in 8.0 mL of dichloromethane. Propionic acid (37 mg, 0.499 mmol) was placed therein, and the mixture was stirred at room temperature for 3 hours, then diluted with chloroform, and washed with water and a saturated sodium chloride solution. The organic layer was dried over anhydrous magnesium sulfate, followed by filtration and distillation of the solvent under reduced pressure. The resulting residue was purified by silica gel column chromatography (chloroform ---> chloroform/acetone = 5:1), thereby obtaining compound SNC3DC (91.4 mg, 41%) as a pale yellow solid.
¹H-NMR (CDCl₃, 400 MΣ3z) : δ = 0.945 (3H, t, J = 7.6 Hz), (3H, t, J = 7.6 Hz), 1.39 (3H, t, J = 7.6 Hz), 2.03-2.12 (1H, m), 2.16-2.25 (1H, m), 2.69 (2H, q, J = 7.5 Hz), 2.78 (2H, t, J = 6.2 Hz), 3.14 (2H, q, J = 7.7 Hz), 3.87-4.02 (2H, m), 5.25 (2H, d, J = 4.8 Hz), 5.33 (1H, d, J = 17.2 Hz), 5.78 (1H, d, J = 17.2 Hz), 7.20 (1H, s), 7.54 (1H, dd, J = 9.0, 2.4 Hz), 7.82 (1H, d, J = 2.4 Hz), 8.16 (1H, d, J = 8.8 Hz).

### SNC4DC

The title compound was obtained by the same production method as for SNC3DC, except for using SNC0DC (500 mg, 0.504 mmol) and butyric acid (110 mg, 1.25 mmol) in place of SN0DC (200 mg, 0.202 mmol) and propionic acid (37 mg, 0.499 mmol). Yield: 62%.
¹H-NMR (CDCl₃, 400 MΣ3z) : δ = 0.958 (3H, t, J = 7.4 Hz), 1.10 (3H, t, J = 7.4 Hz), 1.40 (3H, t, J = 7.4 Hz), 1.81-1.90 (2H, m), 2.05-2.12 (1H, m), 2.17-2.24 (1H, m), 2.65 (2H, t, J = 7.4 Hz), 2.78 (2H, t, J = 6.2 Hz), 3.15 (2H, q, J = 7.6 Hz), 3.87-3.93 (1H, m), 3.96-4.02 (1H, m), 5.27 (2H, d, J = 4.8 Hz), 5.30 (1H, d, J = 17.2 Hz), 5.80 (1H, d, J = 17.2 Hz), 7.21 (1H, s), 7.55 (1H, dd, J = 9.2, 2.4 Hz), 7.56 (1H, d, J = 2.4 Hz), 8.17 (1H, d, J = 9.2 Hz).

### SNC8DC

The title compound was obtained by the same production method as for SNC3DC, except for using SNC0DC (100 mg, 0.101 mmol) and octanoic acid (58 mg, 0.402 mmol) in place of SN0DC (200 mg, 0.202 mmol) and propionic acid (37 mg, 0.499 mmol). Yield: 50%.
¹H-NMR (CDCl₃, 400 MΣ3z) : δ = 0.905 (3H, t, J = 6.8 Hz), 0.957 (3H, t, J = 7.4 Hz), 1.31-1.42 (11H, m), 1.77-1.83 (2H, m), 2.05-2.24 (2H, m), 2.66 (2H, t, J = 7.6 Hz), 2.78 (2H, t, J = 6.2 Hz), 3.15 (2H, q, J = 7.7 Hz), 3.88-4.00 (2H, m), 5.26 (2H, d, J = 5.2 Hz), 5.35 (1H, d, J = 17.2 Hz), 5.80 (1H, d, J = 17.2 Hz), 7.21 (1H, s), 7.55 (1H, dd, J = 9.2, 2.4 Hz), 7.82 (1H, d, J = 2.8 Hz), 8.17 (1H, d, J = 9.2 Hz) .

### SNC10DC

The title compound was obtained by the same production method as for SNC3DC, except for using SNC0DC (103.2 mg, 0.104 mmol) and capric acid (52.1 mg, 0.302 mmol) in place of SN0DC (200 mg, 0.202 mmol) and propionic acid (37 mg, 0.499 mmol).
Yield: 59%.
¹H-NMR (CDCl₃, 400 MΣ3z) : δ = 0.873 (3H, t, J = 7.0 Hz), 0.944 (3H, t, J = 7.6 Hz), 1.27-1.5 (15H, m), 1.76-1.82 (2H, m), 2.03-2.09 (1H, m), 2.17-2.23 (1H, m), 2.65 (2H, t, J = 7.6 Hz), 2.76 (2H, t, J = 6.2 Hz), 3.14 (2H, q, J = 7.7 Hz), 3.85-3.90 (1H, m), 3.93-3.98 (1H, m), 5.24 (2H, d, J = 5.2 Hz), 5.33 (1H, d, J = 17.2 Hz), 5.79 (1H, d, J = 17.2 Hz), 7.19 (1H, s), 7.53 (1H, dd, J = 9.2, 2.4 Hz), 7.80 (1H, d, J = 2.4 Hz), 8.15 (1H, d, J = 9.2 Hz) .

### SNC5DC- (A)

The title compound was obtained by the same production method as for SNC3DC, except for using SNC0DC (200 mg, 0.202 mmol) and 1-methylcyclopropanecarboxylic acid (50 mg, 0.499 mmol) in place of SN0DC (200 mg, 0.202 mmol) and propionic acid (37 mg, 0.499 mmol). Yield: 65%.
¹H-NMR (CDCl₃, 400 MΣ3z) : δ = 0.941-0.960 (5H, m), 1.38 (3H, t, J = 7.7 Hz), 1.48-1.51 (5H, m), 2.02-2.24 (2H, m), 2.77 (2H, t, J = 6.2 Hz), 3.13 (2H, q, J = 7.6 Hz), 3.86-4.01 (2H, m), 5.24 (2H, d, J = 4.4 Hz), 5.33 (1H, d, J = 17.2 Hz), 5.77 (1H, d, J = 17.2 Hz), 7.19 (1H, s), 7.51 (1H, dd, J = 9.2, 2.4 Hz), 7.78 (1H, d, J = 2.4 Hz), 8.14 (1H, d, J = 9.2 Hz).

### SNC5DC- (B)

The title compound was obtained by the same production method as for SNC3DC, except for using SNC0DC (200 mg, 0.202 mmol) and 2-methylcyclopropane-1-carboxylic acid (50 mg, 0.499 mmol) in place of SN0DC (200 mg, 0.202 mmol) and propionic acid (37 mg, 0.499 mmol). Yield: 71%.
¹H-NMR (CDCl₃, 400 MΣ3z) : δ = 0.914-0.961 (4H, m), 1.23 (3H, d, J = 5.6 Hz), 1.31-1.41 (5H, m), 1.62-1.65 (1H, m), 2.04-2.23 (2H, m), 2.77 (2H, t, J = 6.2 Hz), 3.13 (2H, q, J = 7.6 Hz), 3.88-3.99 (2H, m), 5.23 (2H, d, J = 4.4 Hz), 5.33 (1H, d, J = 17.1 Hz), 5.77 (1H, d, J = 17.1 Hz), 7.19 (1H, s), 7.54 (1H, dd, J = 9.2, 2.4 Hz), 7.81 (1H, d, J = 2.4 Hz), 8.14 (1H, d, J = 9.2 Hz).

### SNC5DC- (C)

The title compound was obtained by the same production method as for SNC3DC, except for using SNC0DC (200 mg, 0.202 mmol) and (R)-2-methylbutanoic acid (51 mg, 0.499 mmol) in place of SN0DC (200 mg, 0.202 mmol) and propionic acid (37 mg, 0.499 mmol). Yield: 71%.
¹H-NMR (CDCl₃, 400 MΣ3z) : δ = 0.943 (3H, t, J = 7.4 Hz), 1.07 (3H, t, J = 7.4 Hz), 1.34-1.40 (6H, m), 1.67-1.72 (1H, m), 1.86-1.91 (1H, m), 2.06-2.21 (2H, m), 2.17-2.78 (3H, m), 3.14 (2H, q, J = 7.7 Hz), 3.86-3.96 (2H, m), 5.24 (2H, d, J = 5.2 Hz), 5.33 (1H, d, J = 17.1 Hz), 5.77 (1H, d, J = 17.1 Hz), 7.19 (1H, s), 7.52 (1H, dd, J = 9.2, 2.4 Hz), 7.79 (1H, d, J = 2.4 Hz), 8.15 (1H, d, J = 9.2 Hz).

### SNC5DC-(D)

The title compound was obtained by the same production method as for SNC3DC, except for using SNC0DC (200 mg, 0.202 mmol) and (S)-2-methylbutanoic acid (51 mg, 0.499 mmol) in place of SN0DC (200 mg, 0.202 mmol) and propionic acid (37 mg, 0.499 mmol). Yield: 58%.
¹H-NMR (CDCl₃, 400 MΣ3z) : δ = 0.944 (3H, t, J = 7.5 Hz), 1.07 (3H, t, J = 7.4 Hz), 1.34-1.40 (6H, m), 1.67-1.72 (1H, m), 1.86-1. 93 (1H, m), 2.06-2.21 (2H, m), 2.17-2.78 (3H, m), 3.14 (2H, q, J = 7.7 Hz), 3.87-3.99 (2H, m), 5.24 (2H, d, J = 5.2 Hz), 5.33 (1H, d, J = 17.2 Hz), 5.77 (1H, d, J = 17.2 Hz), 7.19 (1H, s), 7.53 (1H, dd, J = 9.2, 2.4 Hz), 7.79 (1H, d, J = 2.4 Hz), 8.15 (1H, d, J = 9.2 Hz).

### SNC5DC- (E)

The title compound was obtained by the same production method as for SNC3DC, except for using SNC0DC (200 mg, 0.202 mmol) and pentanoic acid (51 mg, 0.499 mmol) in place of SN0DC (200 mg, 0.202 mmol) and propionic acid (37 mg, 0.499 mmol). Yield: 58%.
¹H-NMR (CDCl₃, 400 MΣ3z) : δ = 0.929-1.01 (6H, m), 1.38 (3H, t, J = 7.7 Hz), 1.46-1.51 (2H, m), 1.75-1.83 (2H, m), 2.06-2.21 (2H, m), 2.65 (2H, t, J = 7.4 Hz), 2.77 (2H, t, J = 6.2 Hz), 3.14 (2H, q, J = 7.7 Hz), 3.87-3.99 (2H, m), 5.24 (2H, d, J = 5.2 Hz), 5.33 (1H, d, J = 17.1 Hz), 5.78 (1H, d, J = 17.1 Hz), 7.19 (1H, s), 7.53 (1H, dd, J = 9.2, 2.4 Hz), 7.80 (1H, d, J = 2.4 Hz), 8.15 (1H, d, J = 8.8 Hz).

### SNC5DC- (F)

The title compound was obtained by the same production method as for SNC3DC, except for using SNC0DC (200 mg, 0.202 mmol) and 3-methylbutanoic acid (51 mg, 0.499 mmol) in place of SN0DC (200 mg, 0.202 mmol) and propionic acid (37 mg, 0.499 mmol). Yield: 56%.
¹H-NMR (CDCl₃, 400 MΣ3z) : δ = 0.945 (3H, t, J = 7.5 Hz), 1.10 (6H, d, J = 6.7 Hz), 1.38 (3H, t, J = 7.7 Hz), 2.04-2.33 (3H, m), 2.53 (2H, d, J = 7.1 Hz), 2.77 (2H, t, J = 6.2 Hz), 3.13 (2H, q, J = 7.7 Hz), 3.85-4.01 (2H, m), 5.24 (2H, d, J = 5.2 Hz), 5.33 (1H, d, J = 17.2 Hz), 5.78 (1H, d, J = 17.2 Hz), 7.19 (1H, s), 7.53 (1H, dd, J = 9.2, 2.4 Hz), 7.79 (1H, d, J = 2.4 Hz), 8.15 (1H, d, J = 9.2 Hz).

### SNC5DC- (G)

The title compound was obtained by the same production method as for SNC3DC, except for using SNC0DC (200 mg, 0.202 mmol) and pivalic acid (51 mg, 0.499 mmol) in place of SN0DC (200 mg, 0.202 mmol) and propionic acid (37 mg, 0.499 mmol). Yield: 26%.
¹H-NMR (CDCl₃, 400 MΣ3z) : δ = 0.960 (3H, t, J = 7.5 Hz) ,1.40 (3H, t, J = 7.7 Hz), 1.44 (9H, s), 2.08-2.19 (2H, m), 2.78 (2H, t, J = 6.3 Hz), 3.16 (2H, q, J = 7.7 Hz), 3.90-4.01 (2H, m), 5.26 (2H, d, J = 5.0 Hz), 5.34 (1H, d, J = 17.2 Hz), 5.78 (1H, d, J = 17.2 Hz), 7.21 (1H, s), 7.51 (1H, dd, J = 9.2, 2.4 Hz), 7.79 (1H, d, J = 2.4 Hz), 8.17 (1H, d, J = 9.2 Hz).

### SNC5DC- (H)

The title compound was obtained by the same production method as for SNC3DC, except for using SNC0DC (200 mg, 0.202 mmol) and 2-cyclopropylacetic acid (50 mg, 0.499 mmol) in place of SN0DC (200 mg, 0.202 mmol) and propionic acid (37 mg, 0.499 mmol). Yield: 48%.
¹H-NMR (CDCl₃, 400 MHz): δ = 0.330 (2H, q, J = 5.1 Hz), 0.690 (2H, q, J = 5.1 Hz), 0.980 (3H, t, V J = 6.8 Hz), 1.21-1.25 (1H, m), 1.40 (3H, t, J = 7.7 Hz), 2.05-2.25 (2H, m), 2.57 (2H, d, J = 7.1 Hz), 2.79 (2H, t, J = 6.2 Hz), 3.16 (2H, q, J = 7.7 Hz), 3.89-4.01 (2H, m), 5.27 (2H, d, J = 5.4 Hz), 5.35 (1H, d, J = 17.2 Hz), 5.79 (1H, d, J = 17.2 Hz), 7.21 (1H, s), 7.57 (1H, dd, J = 9.2, 2.4 Hz), 7.85 (1H, d, J = 2.4 Hz), 8.18 (1H, d, J = 9.2 Hz).

### SNC5DC-(I)

The title compound was obtained by the same production method as for SNC3DC, except for using SNC0DC (200 mg, 0.202 mmol) and cyclobutanecarboxylic acid (50 mg, 0.499 mmol) in place of SN0DC (200 mg, 0.202 mmol) and propionic acid (37 mg, 0.499 mmol). Yield: 65%.
¹H-NMR (CDCl₃, 400 MHz): δ = 0.945 (3H, t, J = 7.5 Hz), 1.38 (3H, t, J = 7.7 Hz), 2.04-2.19 (4H, m), 2.37-2.52 (4H, m), 2.77 (2H, t, J = 6.2 Hz), 3.13 (2H, q, J = 7.6 Hz), 3.45-3.49 (1H, m), 3.91-3.97 (2H, m), 5.23 (2H, d, J = 4.6 Hz), 5.33 (1H, d, J = 17.2 Hz), 5.77 (1H, d, J = 17.2 Hz), 7.19 (1H, s), 7.53 (1H, dd, J = 9.2, 2.4 Hz), 7.81 (1H, d, J =2.4 Hz), 8.15 (1H, d, J = 9.2 Hz) .

### Example 7

### Method for producing compounds SNC3NDC and SNC4NDC of the present invention

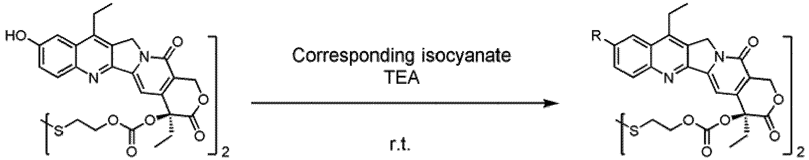

First, the production method is described using SNC3NDC as an example.

SN0DC (263.9 mg, 0.266 mmol), 1-isocyanatopropane (64.3 mg, 0.756 mmol), and triethylamine (127.5 mg, 1.26 mmol) were placed in an eggplant flask equipped with a stirrer bar, and dissolved in 5.0 mL of dichloromethane. After stirring at room temperature for 4 hours, the resultant was diluted with chloroform and washed with water, a saturated ammonium chloride aqueous solution, and a saturated sodium chloride solution. The organic layer was dried over anhydrous magnesium sulfate, followed by filtration and distillation of the solvent under reduced pressure. The resulting residue was purified by silica gel column chromatography (chloroform ---> chloroform/acetone = 5:1), thereby obtaining compound SNC3NDC (92.8 mg, 30%) as a pale yellow solid.
¹H-NMR (CDCl₃, 400 MHz): δ = 0.951-1.03 (6H, m), 1.37 (3H, t, J = 7.6 Hz), 1.63-1.68 (2H, m), 2.79 (2H, t, J = 6.2 Hz), 3.11 (2H, q, J = 6.8 Hz), 3.29 (2H, q, J = 6.8 Hz), 3.94-3.97 (1H, m), 4.01-4.06 (1H, m), 5.22 (2H, d, J = 1.6 Hz), 5.32-5.37 (2H, m), 5.77 (1H, d, J = 17.2 Hz), 7.20 (1H, s), 7.56 (1H, dd, J = 9.2, 2.4 Hz), 7.84 (1H, d, J = 2.4 Hz), 8.12 (1H, d, J = 9.2 Hz).

### SNC4NDC

The title compound was obtained by the same production method as for SNC3NDC, except for using SNC0DC (255.4 mg, 0.258 mmol) and 1-isocyanatobutane (75 mg, 0.757 mmol) in place of SN0DC (263.9 mg, 0.266 mmol) and 1-isocyanatopropane (64.3 mg, 0.756 mmol). Yield: 19%.
¹H-NMR (CDCl₃, 400 MHz) : δ = 0.934-0.990 (6H, m), 1.34-1.46 (5H, m), 1.57-1.64 (2H, m), 2.05-2.10 (1H, m), 2.17-2.24 (1H, m), 2.79 (2H, t, J = 7.0 Hz), 3.07-3.13 (2H, m), 3.29-3.34 (2H, q, J = 6.6 Hz), 3.94-4.05 (2H, m), 5.21 (2H, d, J = 1.6 Hz), 5.32-5.36 (2H, m), 5.76 (1H, d, J = 17.2 Hz), 7.20 (1H, s), 7.55 (1H, dd, J = 9.2, 2.4 Hz), 7.83 (1H, d, J = 2.4 Hz), 8.11 (1H, d, J = 9.2 Hz).

### Example 8

### Production of SNCnDC (n = 0, 2, 4, 6, 8, or 10) nanoparticles

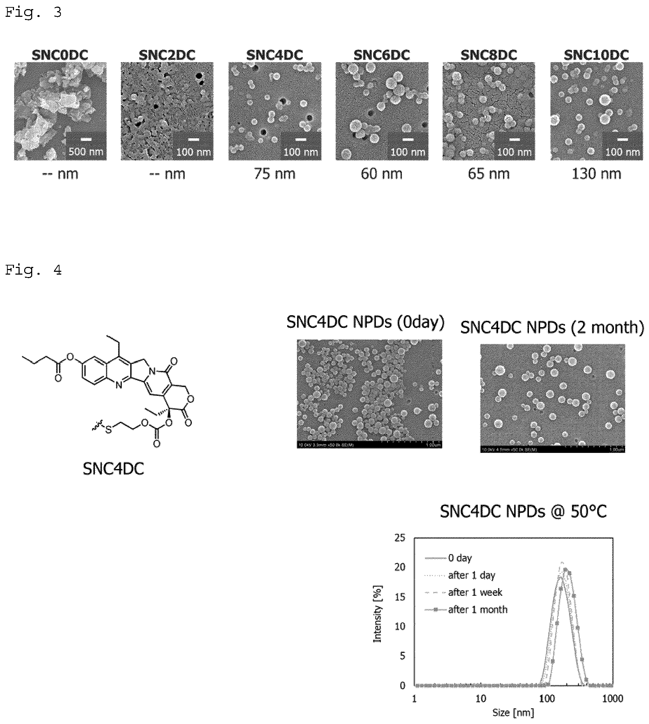

100 µL of DMSO or THF solution of each compound prepared to 10 mM was injected into 10 mL of water using a syringe at room temperature, followed by stirring for 2 seconds at 1500 rpm, thereby obtaining an aqueous dispersion of nanoparticles. The concentration of the final aqueous dispersion was 0.1 mM. SEM and DLS revealed that when n = 0 or 2, the nanoparticles aggregated immediately. It was revealed that when n = 4, 6, 8, or 10, the particle size was about 100 nm. Fig. 3 shows the SEMs of the nanoparticles of the compounds obtained above.

### Example 9

### Method for producing high-concentration nanoparticles

750 µL of THF solution of compound SNC4DC prepared to 46.7 mM was injected into 4.25 mL of water using a syringe at room temperature, thereby obtaining an aqueous dispersion of nanoparticles. The concentration of the final aqueous dispersion was 7 mM. When the THF solution was prepared, the same amount of polysorbate 80 as the compound was added. After removing THF, the resultant was quantified again to 5 mL and mixed with 9% sodium chloride solution at a ratio of 9:1 to prepare the nanoparticles. The results of SEM images of the nanoparticles immediately after the preparation of the high-concentration dispersion and two months after the preparation are shown in the upper-right photographs in Fig. 4. The results of changes in the particle size distribution of the nanoparticles over time are shown in the lower-right graph in Fig. 4.

### Test Example 2

### In vitro activity test

As in Test Example 1, human colon cancer cell line HCT-116 was seeded in a 96-well plate at 2×10⁴ cells/well. On the next day, SN-38 and each of the SN-38 derivative nanoparticle dispersions were added to the cell culture medium to 0.04 to 10 µM. Then, the cells were cultured for 48 hours, and the cell viability was measured by a colorimetric method using the Cell Counting Kit-8 (produced by Dojindo Laboratories) and a microplate reader. Table 2 below shows the results.

**Table 2**

| Compound | IC₅₀ [µM] | Compound | IC₅₀ [µM] |
|---|---|---|---|
| SNC0DC | 0.15±0.016 | SNC5DC-(A) | 0.37±0.054 |
| SNC2DC | 0.18±0.072 | SNC5DC-(B) | 0.34±0.031 |
| SNC3DC | 0.06±0.002 | SNC5DC-(C) | 0.27±0.043 |
| SNC4DC | 0.08±0.006 | SNC5DC-(D) | 0.18±0.019 |
| SNC6DC | 0.55±0.094 | SNC5DC-(E) | 0.20±0.019 |
| SNC8DC | 1.21±0.271 | SNCSDC-(F) | 0.20±0.026 |
| SNC10DC | 1.52±0.301 | SNCSDC-(G) | 0.26±0.028 |
| SNC3NDC | 0.10±0.015 | SNC5DC-(H) | 0.09±0.009 |
| SNC4NDC | 0.08±0.007 | SNC5DC-(I) | 0.20±0.044 |
| SNBocDC | 0.30±0.088 | | |

As shown in Table 2 above, each of the compounds of the present invention showed different IC50 values depending on the substituent. Based on the above results, SNC4DC, which had the highest pharmacological activity among the nanoparticles produced in Fig. 3, was used to perform Test Examples 3 and 4 shown below.

### Test Example 3

### In vivo antitumor activity test

A total of 100 µL was administered by intravenous injection every 2 days for a total of 5 times to mice (n=5) carrying HCT116 cells derived from human colon cancer under the conditions shown in Table 3 below.

**Table 3**

| Experimental method |
|---|
| Cancer cells: HCT-116 cells (derived from human colon cancer) |
| Cell density: 5.0×10⁶ cells/100 µL |
| Injection site: both hind leg bases |
| Anticancer drug administration: start administration after the tumor size reaches about 3 to 5 mm; once every 2 days for a total of 5 times |
| Type of anticancer drug: irinotecan, SNC4DC NPDs |
| Dose: 10 mg/kg/day (in terms of SN-38) |

In the table, "SNC4DC NPDs" refer to the nanoparticles of SNC4DC obtained in Example 9.

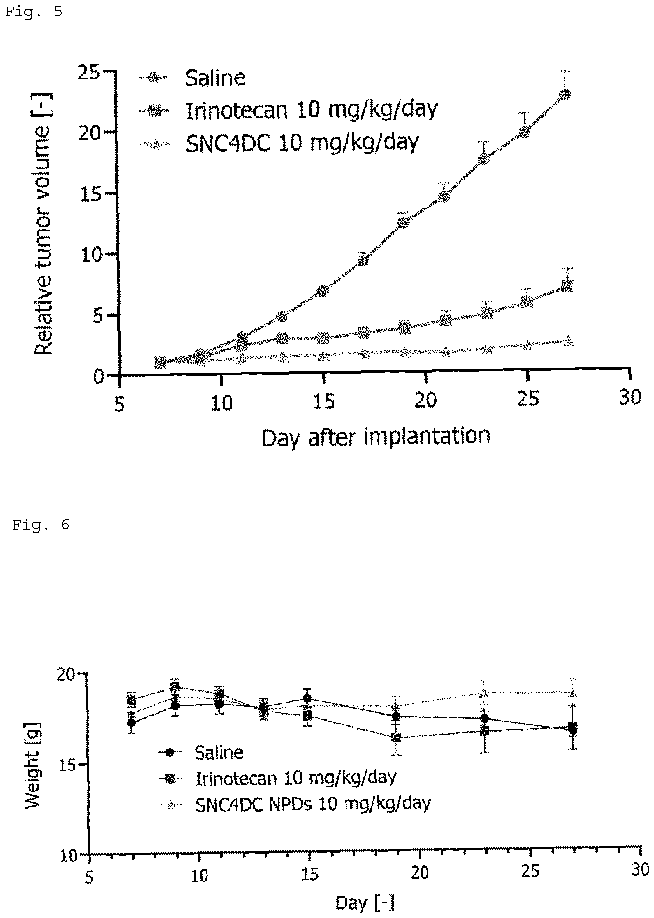

The tumor size was measured using calipers. Fig. 5 shows the results of the in vivo antitumor activity test. As shown in Fig. 5, SNC4DC NPDs exhibited higher antitumor activity than irinotecan. Further, Fig. 6 shows changes in body weight over time in each test group. As shown in Fig. 6, no significant weight loss was observed following the administration of SNC4DC NPDs. Therefore, no serious toxicity due to the administration of SNC4DC NPDs was observed.

### Test Example 4

### In vitro activity test

As in Test Example 1, human colon cancer cell line HCT-116 or human mammary epithelial cell line HMEC was seeded in a 96-well plate at 2×10⁴ cells/well. On the next day, SN-38 and the SN-38 derivative (SNC4DC) nanoparticle dispersion were added to the cell culture medium to 0.04 to 10 µM. Then, the cells were cultured for 48 hours, and the cell viability was measured by a colorimetric method using the Cell Counting Kit-8 (produced by Dojindo Laboratories) and a microplate reader.

Table 4 below shows the results of cell viability IC50.

**Table 4**

| **Cell line** | **HCT-116 (Cancer cell)** | | **HMEC (Normal cell)** | |
|---|---|---|---|---|
| **Compounds** | **SN-38** | **SNC4DC** | **SN-38** | **SNC4DC** |
| **IC₅₀ [µM]** | **0.04** | **0.06** | **0.55** | **11.6** |

As shown in Table 4, SN-38 was highly toxic to normal cells, whereas SNC4DC was less toxic to normal cells.

## Claims

1. A compound represented by the following formula (1) or a salt thereof: 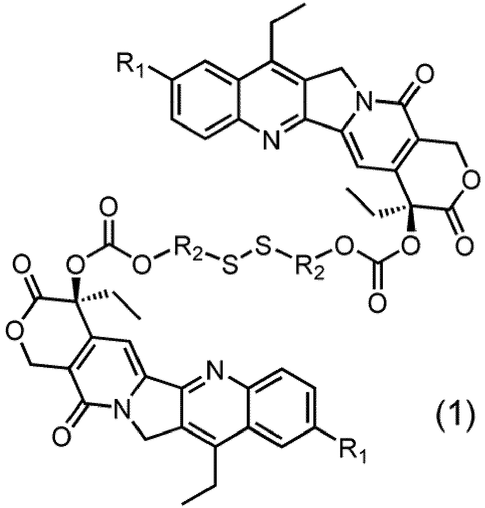 wherein R¹s are the same or different and each represent hydrogen, a hydroxy group, or a carbon-containing organic group; and R²s are the same or different and each represent an optionally substituted ethylene group or trimethylene group.

2. The compound according to claim 1 or a salt thereof, which has a ClogP of 5.0 to 14.0.

3. The compound according to claim 1 or 2 or a salt thereof, wherein R¹ is hydrogen, a hydroxy group, -O-C(=O)-L-R³, wherein L represents a single bond or a linker group, and R³ represents an optionally substituted unsaturated hydrocarbon ring group or heterocyclic group, or -O-C(=O)-(Y)n-R⁴, wherein Y represents a nitrogen atom or an oxygen atom, n represents 0 or 1, and R⁴ represents a C2-C10 alkyl group, a C3-C10 cycloalkyl group, a C4-C10 cycloalkyl-alkyl group, or a C4-C10 alkyl-cycloalkyl group.

4. Nanoparticles comprising the compound according to any one of claims 1 to 3 or a salt thereof.

5. A medicine comprising the compound according to any one of claims 1 to 3 or a salt thereof, or the nanoparticles according to claim 4.

6. The medicine according to claim 5, for use in prevention or treatment of cancer.

7. A method for producing nanoparticles, comprising injecting a water-miscible organic solvent solution of the compound according to any one of claims 1 to 3 or a salt thereof into water.
